# EUROPEAN PATENT SPECIFICATION

(11) **EP 3 705 040 B1**
(45) Date of publication and mention of the grant of the patent: **28.10.2026**
(21) Application number: 18874775.2
(22) Date of filing: 04.09.2018
(51) Int. Cl.: G06F 18/00, A61B 5/117, A61B 5/11

(54) **WALKING VIBRATION ANALYSIS DEVICE, WALKING VIBRATION ANALYSIS METHOD, AND COMPUTER-READABLE RECORDING MEDIUM**
VORRICHTUNG ZUR ANALYSE VON LAUFSCHWINGUNGEN, VERFAHREN ZUR LAUFSCHWINGUNGSANALYSE UND COMPUTERLESBARES AUFZEICHNUNGSMEDIUM
DISPOSITIF D'ANALYSE DES VIBRATIONS LORS DE LA MARCHE, MÉTHODE D'ANALYSE DES VIBRATIONS LORS DE LA MARCHE ET SUPPORT D'ENREGISTREMENT LISIBLE PAR ORDINATEUR"

(30) Priority: 02.11.2017 JP 2017212737
(43) Date of publication of application: 09.09.2020
(73) Proprietor: NEC Solution Innovators, Ltd., Tokyo 136-8627 (JP)
(72) Inventor: SHIMIZU, Hideyuki, Tokyo 136-8627 (JP)
(74) Representative: Vossius & Partner Patentanwälte Rechtsanwälte mbB
(86) International application number: PCT/JP2018/032779
(87) International publication number: WO 2019/087563

(56) References cited:
- EP-A1- 3 099 229
- WO-A1-2016/130571
- WO-A1-2017/188418
- JP-A- 2001 190 527
- JP-A- 2004 227 053
- JP-A- 2006 110 072
- JP-A- 2011 164 993
- JP-A- 2012 168 647
- JP-A- 2015 139 667
- JP-A- 2015 139 667
- US-A1- 2010 070 187
- US-A1- 2016 307 031
- NAKAMURA YUICHI ET AL.: "Individual Identification by Gait Vibration Data Transmitted Floor,", AUTOMATION AND SYSTEMS - 2016 16TH INTERNATIONAL CONFERENCE ON CONTROL, 26 January 2017 (2017-01-26), pages 1371 - 1376, XP033051737

## Description

### TECHNICAL FIELD

The example embodiments relates to a walking vibration analysis apparatus and a walking vibration analysis method that are for analyzing vibration that occurs due to walking, and furthermore relates to a computer-readable recording medium that includes a program recorded thereon for realizing the apparatus and method.

### BACKGROUND ART

In recent years, systems that install sensors in a residence for purposes such as watching over elderly people when they are at home have been proposed. With such systems, it is necessary to both protect the privacy of the resident and identify the resident. Thus, in view of protecting privacy, methods for identifying a resident using a vibration sensor rather than using a camera sensor have been proposed. That is, such methods perform identification by extracting a feature of an individual from vibration associated with walking (hereinafter, "walking vibration") measured with a vibration sensor installed in the residence (e.g., see Patent Document 1 and Non-Patent Document 1).

Specifically, Patent Document 1 discloses a system that specifies an individual from walking vibration. In the system disclosed in Patent Document 1, a frequency of vibration produced due to an action of a person within a space or a period of multiple steps is extracted as a feature amount of an individual from measured walking vibration, and collated with frequencies or periods of individuals collected in advance to specify an individual.

Non-Patent Document 1 also discloses a system that specifies an individual from walking vibration. The system disclosed in Non-Patent Document 1 specifies an individual by utilizing the fact that vibration patterns differ between individuals.
US 2010/070187 A1 relates to a human recognition apparatus and a human recognition method identifying a user based on a walking pattern. Documents WO 2016/130571 A1 (UNIV CARNEGIE MELLON [US]) 18 August 2016, EP 3 099 229 A1 (KONINKLIJKE PHILIPS NV [NL]) 7 December 2016 and US 2016/307031 A1 (ZHONG YU [US] ET AL) 20 October 2016, represent further prior art.

### LIST OF RELATED ART DOCUMENTS

### PATENT DOCUMENT

Patent Document 1: Japanese Patent Laid-Open Publication No. 2004-227053

### NON-PATENT DOCUMENT

Non-Patent Document 1: Kazuki ITO et al., "Construction of Individual Identification System using Gait Vibration Data" [online], Symposium on the Living Body, Sensibility and Advanced Information Processing, 2017 [viewed on August 31, 2015], Internet URL: http://pelican.nagaokaut.ac.jp/2017symposium/pdf/09-S-1%E3%80%80%E4%BC%8A%E8%97%A4%E3%80%80%E5%92%8C%E8%BC%9D%EF% BC%88%E9%98%BF%E5%8D%97%E5%B7%A5%E6%A5%AD%E9%AB%98%E7%AD%8 9%E5%B0%82%E9%96%80%E5%AD%A6%E6%A0%A1%EF%BC%89.pdf

### SUMMARY

### PROBLEMS

However, in actual fact, patterns of walking vibration are not constant but vary, even when the same person walks through the same place. Thus, there is a problem in that both the system disclosed in Patent Document 1 and the system disclosed in Non-Patent Document 1 have difficulty in specifying an individual.

An example object of the example embodiments is to solve the above problems and provide a walking vibration analysis apparatus, a walking vibration analysis method, and a computer-readable recording medium that can enable an individual to be specified from walking vibration, even in the case where variation occurs in the walking vibration.

### MEANS FOR SOLVING THE PROBLEMS

The invention is defined in the independent claims. Dependent claims describe preferred embodiments.

### ADVANTAGEOUS EFFECTS

As described above, according to the example embodiments, an individual can be specified from walking vibration, even in the case where variation occurs in the walking vibration.

### BRIEF DESCRIPTION OF THE DRAWINGS

FIG. 1 is a block diagram showing a schematic configuration of a walking vibration analysis apparatus in an example embodiment.
FIG. 2 is a block diagram more specifically showing the configuration of the walking vibration analysis apparatus in the example embodiment.
FIG. 3 is a diagram showing an example of walking vibration data that is acquired in the example embodiment.
FIG. 4 is a diagram showing an example of binary data that is obtained in the example embodiment.
FIG. 5 is a diagram showing an example of sample data that is acquired in the example embodiment.
FIG. 6 is a diagram showing a state where the sample data shown in FIG. 5 has been binarized.
FIG. 7 is a diagram showing an example of composited sample data in the example embodiment.
FIG. 8 is a diagram showing a state after filtering of the sample data shown in FIG. 7.
FIGS. 9 are diagrams showing an example of created high-level data in the example embodiment. FIGS. 9(a) to 9(d) respectively show the high-level data of different individuals.
FIG. 10 is a diagram showing an example of a table that consolidates individual specification data that is created in the example embodiment.
FIG. 11 is a diagram showing an example of binary data targeted for determination in the example embodiment.
FIG. 12(a) is a diagram showing a state where the binary data shown in FIG. 11 and the individual specification data of person A are superimposed, and FIG. 12(b) is a diagram showing a state where the binary data shown in FIG. 11 and the individual specification data of person B are superimposed.
FIG. 13 is a flow diagram showing operations at the time of individual specification data creation processing by the walking vibration analysis apparatus according to the example embodiment.
FIG. 14 is a flow diagram showing operations at the time of individual specification processing by the walking vibration analysis apparatus according to the example embodiment.
FIG. 15 is a block diagram showing an example of a computer that realizes the walking vibration analysis apparatus according to the example embodiment.

### EXAMPLE EMBODIMENTS

### (Example Embodiment)

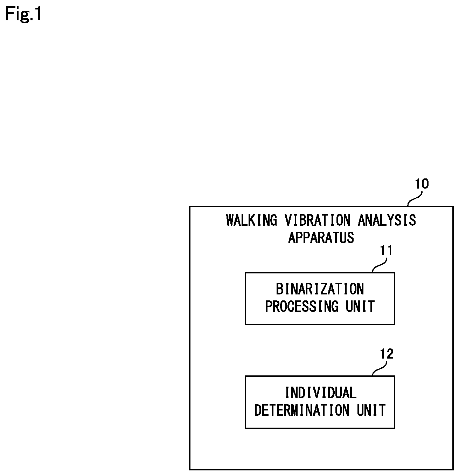

Hereinafter, a walking vibration analysis apparatus according to an example embodiment will be described, with reference to FIGS. 1 to 15.

### [Apparatus configuration]

Initially, a schematic configuration of the walking vibration analysis apparatus according to the example embodiment will be described using FIG. 1. FIG. 1 is a block diagram showing a schematic configuration of the walking vibration analysis apparatus according to the example embodiment.

A walking vibration analysis apparatus 10 according to the example embodiment shown in FIG. 1 is an apparatus for specifying an individual by analyzing vibration that occurs due to walking. As shown in FIG. 1, the walking vibration analysis apparatus 10 includes a binarization processing unit 11 and an individual determination unit 12.

The binarization processing unit 11 acquires walking vibration data specifying walking vibration produced in association with walking, and converts the acquired walking vibration data into binary data. The binary data is data representing the value of vibration over time from the start of walking with two values, namely, high level and low level.

The individual determination unit 12 specifies the individual from which the walking vibration data was acquired, by collating the binary data obtained through conversion with individual specification data. The individual specification data is data created, for each individual in advance, by converting the walking vibration data of the individual into binary data.

In this way, in the example embodiment, the walking vibration analysis apparatus 10 extracts features of an individual in the walking vibration through binarization, and specifies an individual by the extracted features. Thus, according to the example embodiments, an individual can be specified from walking vibration, even in the case where variation occurs in the walking vibration.

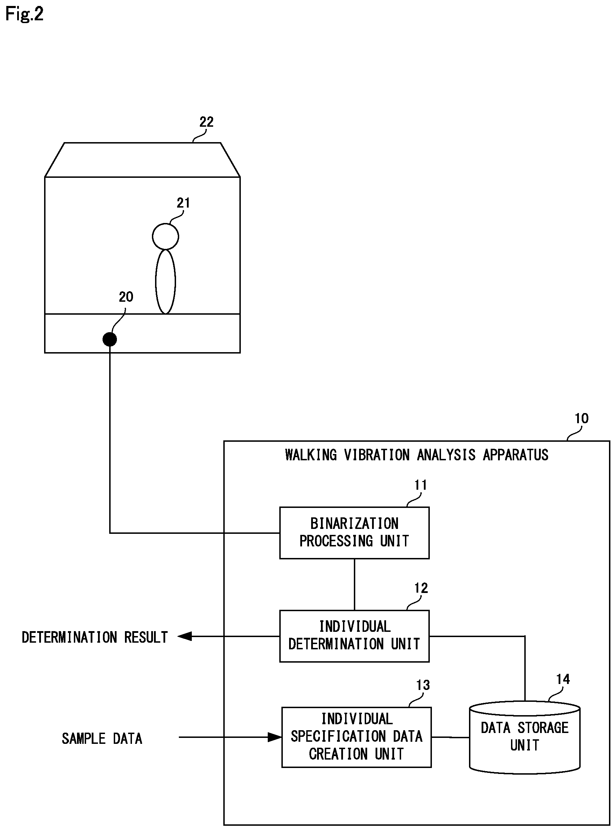

Next, the configuration of the walking vibration analysis apparatus 10 according to the example embodiment will be more specifically described using FIGS. 2 to 12. FIG. 2 is a block diagram more specifically showing the configuration of the walking vibration analysis apparatus according to the example embodiment.

First, in the example embodiment, as shown in FIG. 2, the walking vibration analysis apparatus 10 is connected to a vibration sensor 20 for detecting walking vibration. An acceleration sensor, for example, is used as the vibration sensor 20. Also, the vibration sensor 20 is attached to the floor of a building 22 such as a residence, for example, and detects vibration that occurs due to walking of a person 21 who is in the building 22.

The binarization processing unit 11, first, acquires sensor data output by the vibration sensor 20. The sensor data is walking vibration data specifying walking vibration produced in association with walking. The binarization processing unit 11 then calculates a standard deviation σ of the walking vibration data (sensor data), and converts the walking vibration data into binary data, using the calculated standard deviation σ as a threshold value.

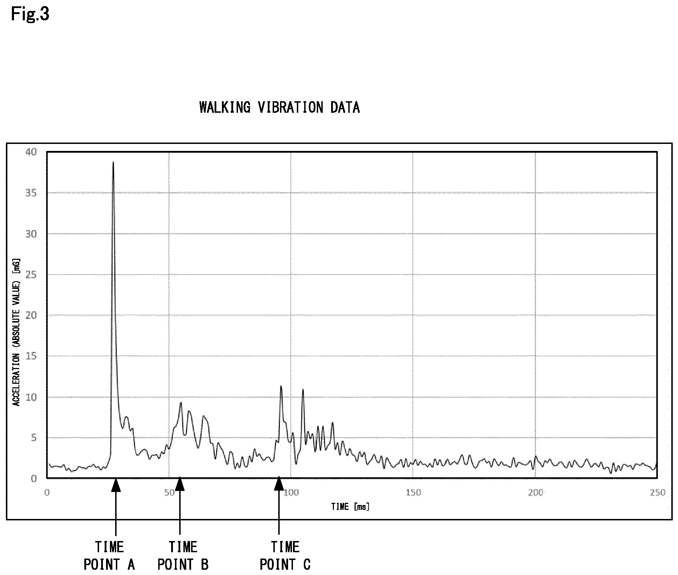

Specifically, the binarization processing unit 11 acquires M steps (M: any natural number) of walking vibration data of a given interval shown in FIG. 3. FIG. 3 is a diagram showing an example of walking vibration data that is acquired in the example embodiment. In the example of FIG. 3, the given interval is the interval of one step, and includes from the point in time (time point A) at which the heel of one foot of the person contacts the floor until the point in time (time point C) at which the toe of the person contacts the floor. Also, in the example in FIG. 3, time point B is the point in time at which the toe of the other foot lifts off the floor. The walking vibration data shown in FIG. 3 is one step of walking vibration data.

Also, in the example in FIG. 3, the sampling rate of the vibration sensor 20 is set to 1 ksps. Furthermore, the number of pieces N of walking vibration data in the given interval is set to 299, and the length is set to 230 ms. Accordingly, the binarization processing unit 11 acquires aₘ₀ to a_{mN} as values of the walking vibration data of the mth step of the given interval, where "aₘₙ" (0 ≤ n ≤ N) is the value of each piece of walking vibration data at any given point in time of the mth step (m = 1 to M).

Next, the binarization processing unit 11 calculates the standard deviation σ, based on the following equation 1, for each piece of acquired walking vibration data (aₘ₀ to a_{mN}). Note that, in the following equation 1, a̅ is the average value of aₘ₀ to a_{mN}. [Equation 1] $σ = \sqrt{\frac{1}{N + 1} {\sum }_{n = 0}^{N} {\left({a}_{mn}-\overline{a}\right)}^{2}}$

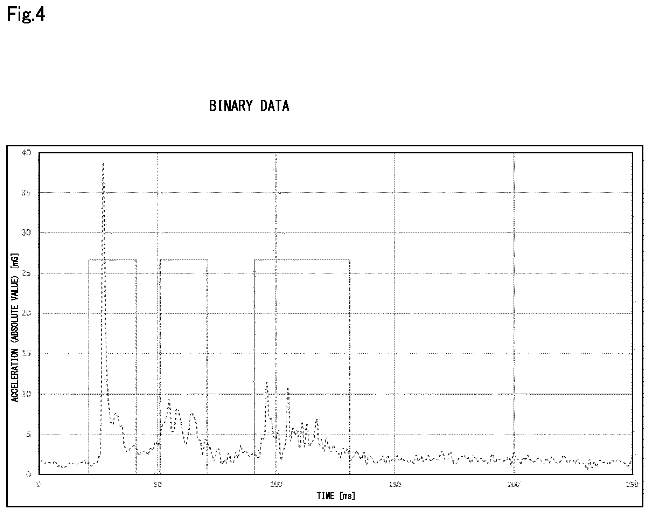

Furthermore, the binarization processing unit 11, as shown in FIG. 4, converts the walking vibration data into binary data, using the calculated standard deviation σ as a threshold value. M steps of walking vibration data are thereby binarized. FIG. 4 is a diagram showing an example of binary data that is obtained in the example embodiment. In the binary data thus obtained, the horizontal axis shows time and the vertical axis shows acceleration (absolute value).

Specifically, the binarization processing unit 11 calculates an average value Av of the walking vibration data every 10 ms, for example, and compares the calculated average value with the standard deviation σ. The binarization processing unit 11, as shown in the following equation 2, then sets the value aₘₙ of the walking vibration data targeted for comparison to "bₘₙ = 0", in the case where the result of comparison indicates that the average value Av is smaller than the standard deviation σ. On the other hand, in the case where the average value Av is greater than or equal to the standard deviation σ, the binarization processing unit 11 sets the value aₘₙ of the walking vibration data targeted for comparison to "bₘₙ = 1". [Equation 2] $\begin{matrix}A v < σ : {a}_{mn} \to {b}_{mn} = 0 \\ A v ≧ σ : {a}_{mn} \to {b}_{mn} = 1\end{matrix}$

Also, as shown in FIG. 2, in the example embodiment, the walking vibration analysis apparatus 10 includes an individual specification data creation unit 13 and a data storage unit 14, in addition to the binarization processing unit 11 and the individual determination unit 12. The individual specification data creation unit 13 creates, for each individual, individual specification data of the individual. Also, the individual specification data creation unit 13 stores the created individual specification data in the data storage unit 14.

Specifically, the individual specification data creation unit 13, first, acquires, for each individual, a plurality of steps of walking vibration data of the individual. The walking vibration data that is acquired at this time is data to be used in creating individual specification data, and will be notated hereinafter as "sample data". Also, the individual specification data creation unit 13 converts, for each individual, the acquired plurality of steps of sample data of the individual into binary data. Note that, at this time, the individual specification data creation unit 13 may perform binarization with similar processing to the binarization processing unit 11, or may instruct the binarization processing unit 11 to binarize the sample data.

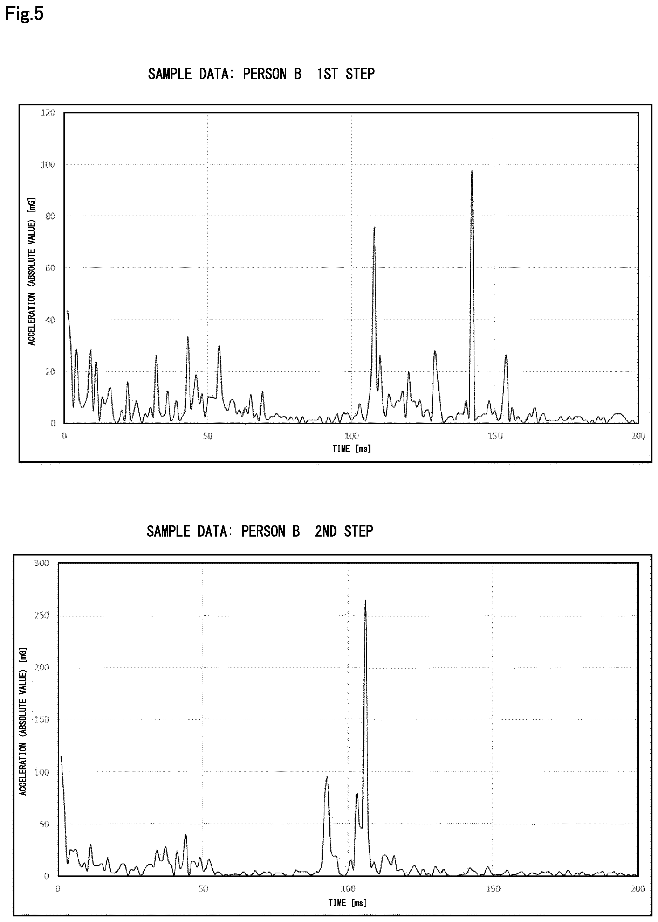

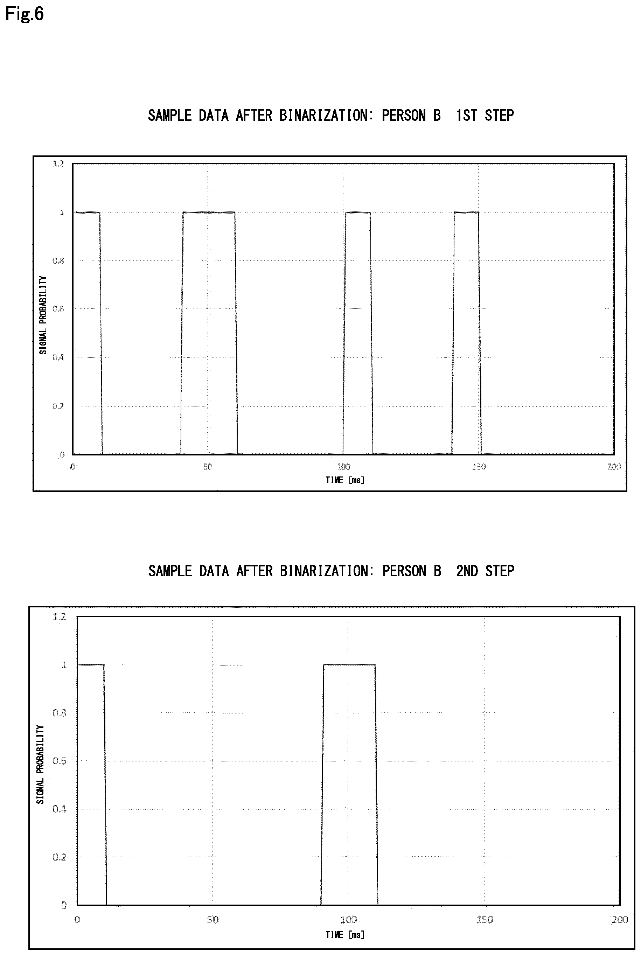

FIG. 5 is a diagram showing an example of sample data that is acquired in the example embodiment. FIG. 6 is a diagram showing a state where the sample data shown in FIG. 5 has been binarized. In the example in FIG. 5, the sample data obtained with the first step and the sample data obtained with the second step of walking by a certain individual are shown. Since these pieces of sample data do not coincide despite having been acquired from the same person, the data will also differ from each other after binarization as shown in FIG. 6. FIG. 6 similarly shows time on the horizontal axis and signal probability on the vertical axis.

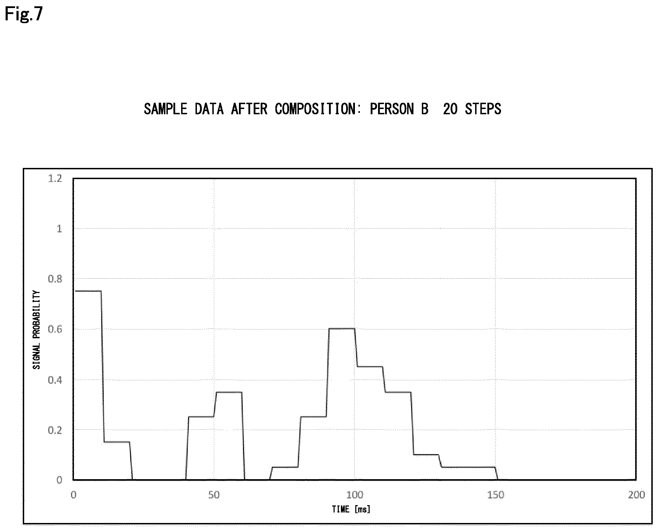

Next, the individual specification data creation unit 13 calculates the probability of occurrence of high-level portions in association with elapsed time from the start of walking, based on the plurality of steps of walking vibration data converted into binary data, and takes the calculated probability of occurrence as the individual specification data of the individual. This point will be described in detail using FIGS. 7 to 10.

As shown in FIG. 7, the individual specification data creation unit 13, first, composites the M steps of sample data that are acquired for a specific individual. Composition is performed by calculating an average value cₙ for every piece of sample data whose n value is the same (value = bₘₙ) using the following equation 3. FIG. 7 is a diagram showing an example of composited sample data in the example embodiment. FIG. 7 similarly shows time on the horizontal axis and signal probability on the vertical axis. [Equation 3] ${c}_{n} = \sum {b}_{mn} / M$

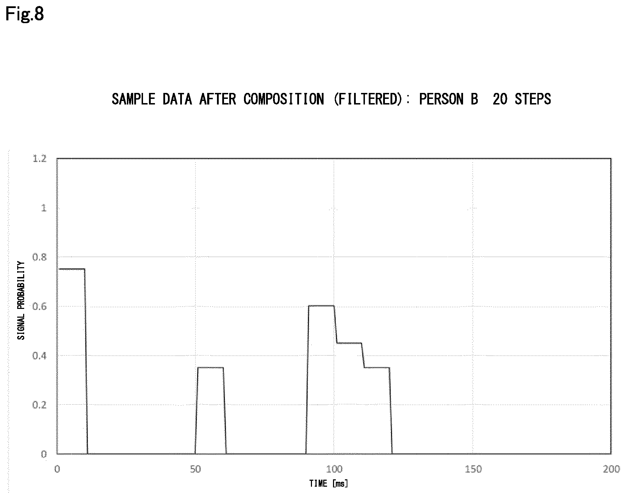

Next, as shown in FIG. 8, the individual specification data creation unit 13 filters the sample data after composition, using a threshold value α shown in the following equation 4. The threshold value α is used for filtering parts that have a low signal probability and possibly contain noise. The value cₙ of the sample data is thereby converted into dₙ based on the threshold value α. FIG. 8 is a diagram showing a state after filtering of the sample data shown in FIG. 7. FIG. 8 similarly shows time on the horizontal axis and signal probability on the vertical axis. In FIG. 8, the threshold value α is set to 0.25. [Equation 4] $\begin{matrix}{c}_{n} ≦ α \to {d}_{n} = 0 \\ {c}_{n} > α \to {d}_{n} = {c}_{n}\end{matrix}$

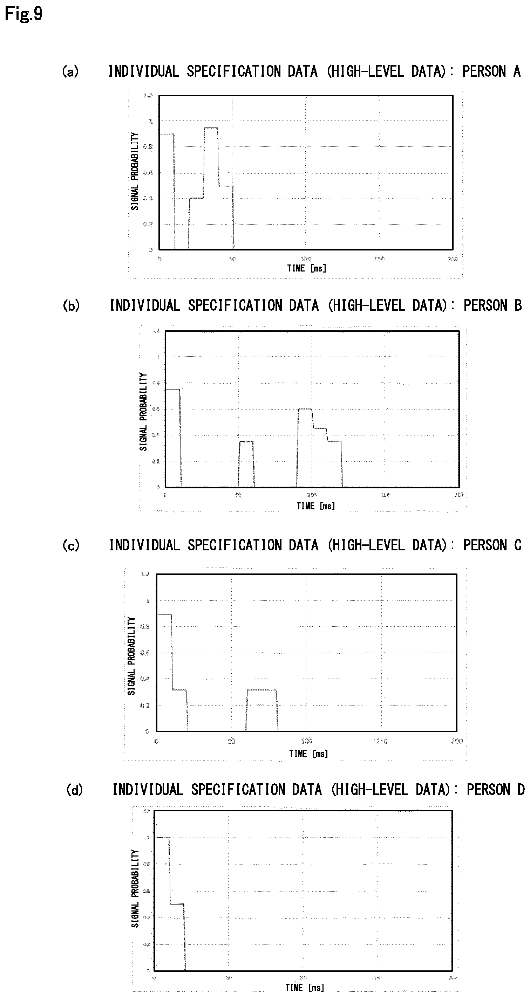

The obtained data after filtering shown in FIG. 8 will indicate the probability of occurrence of high-level portions in the walking vibration data of the specific individual. In other words, in FIG. 8, the non-zero portions indicate portions where high-level portions occur with high probability. Hereinafter, the data shown in FIG. 8 will be notated as "high-level data". Also, high-level data is created for each individual, as shown in FIGS. 9. FIGS. 9 are diagrams showing an example of created high-level data in the example embodiment. FIGS. 9(a) to 9(d) respectively show the high-level data of different individuals.

Also, among the high-level portions respectively shown in FIGS. 9(a) to 9(d), portions corresponding to time point A shown in FIG. 3 will be notated as "peak 1", portions corresponding to time point B shown in FIG. 3 will be notated as "peak 2", and portions corresponding to time point C shown in FIG. 3 will be notated as "peak 3".

It is evident from FIG. 9(a) that, with person A, the probability of occurrence of peaks 1 and 2 is high. Similarly, it is evident from FIG. 9(b) that, with person B, the probability of occurrence of peaks 1, 2 and 3 is high. Also, it is evident from FIG. 9(c) that, with person C, the probability of occurrence of peaks 1 and 3 is high. Furthermore, it is evident from FIG. 9(d) that, with person D, the probability of occurrence of peak 1 is high.

The high-level data, respectively shown in FIGS. 9(a) to 9(d), obtained in this manner will be the individual specification data of each individual. In the individual specification data, the probability of occurrence of high-level portions is associated with elapsed time from the start of walking. In this way, in the example embodiment, individual specification data is created, by superimposing a plurality of steps of walking vibration, and indexing the probability of a high-level portion occurring at respective timings.

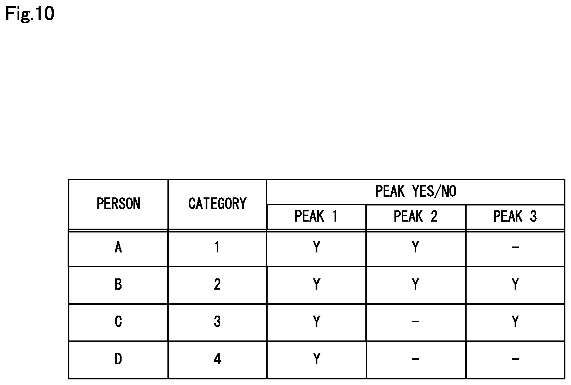

Also, the individual specification data creation unit 13 creates a table shown in FIG. 10 from the individual specification data of each individual. FIG. 10 is a diagram showing an example of a table consolidating individual specification data that is created in the example embodiment. In the table shown in FIG. 10, persons A to D are sorted into one of categories 1 to 4, depending on the combination of peaks with a high probability of occurrence.

Also, in the example embodiment, the individual determination unit 12, first, acquires the individual specification data of each individual created by the individual specification data creation unit 13 from the data storage unit 14. Next, the individual determination unit 12 acquires binary data targeted for determination from the binarization processing unit 11. The individual determination unit 12 then contrasts, for each piece of individual specification data, high-level portions of the acquired binary data with the probability of occurrence of high-level portions of the individual specification data.

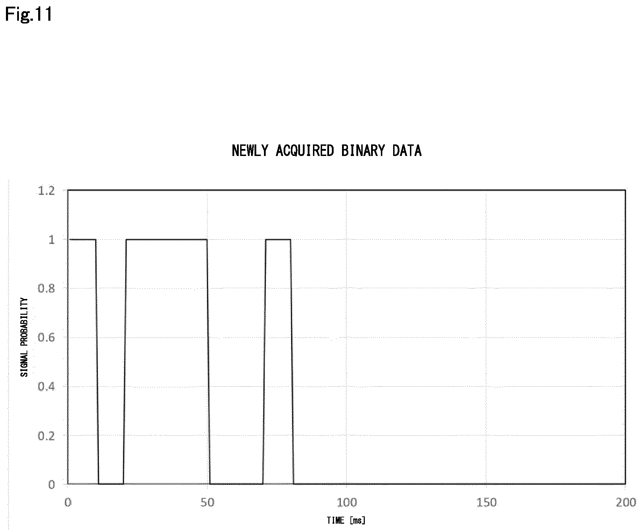

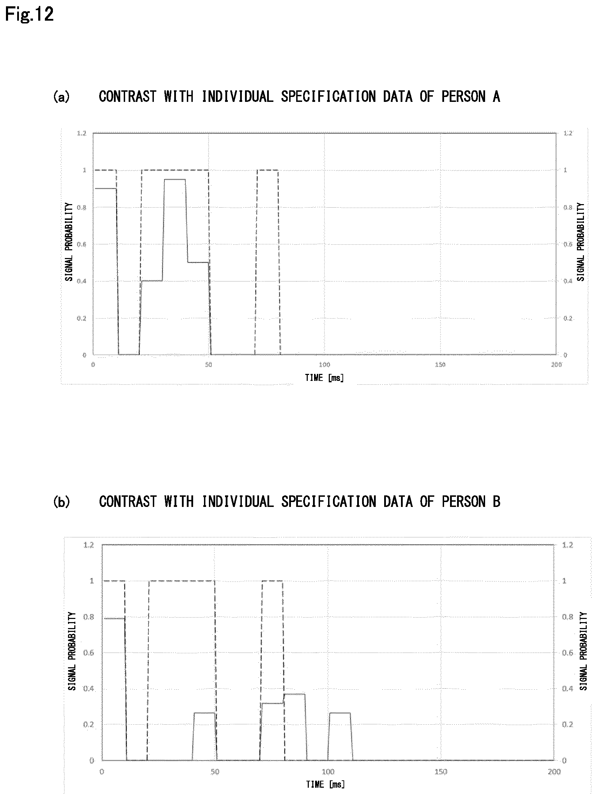

For example, assume that the acquired binary data targeted for determination is the binary data shown in FIG. 11. The result of superimposing the binary data shown in FIG. 11 and the individual specification data shown in FIGS. 9(a) and 9(b) will be as shown in FIGS. 12(a) and 12(b). FIG. 11 is a diagram showing an example of binary data targeted for determination in the example embodiment. FIG. 12(a) is a diagram showing a state where the binary data shown in FIG. 11 and the individual specification data of person A are superimposed, and FIG. 12(b) is a diagram showing a state where the binary data shown in FIG. 11 and the individual specification data of person B are superimposed.

The individual determination unit 12 then calculates, for each piece of individual specification data, the probability of the individual from which the walking vibration data was acquired being the individual corresponding to the individual specification data, based on the results of contrasting the binary data and the individual specification data.

Specifically, the individual determination unit 12, first, calculates the sum of products S of the values (d₀ to d_{N}) of the individual specification data and the values (s₀ to s_{N}) of the binary data, based on the following equation 5, where s₀ to s_{N} are the respective values of the binary data targeted for determination. [Equation 5] $S = \sum \left({d}_{n}\times {s}_{n}\right)$

Next, the individual determination unit 12 normalizes the sum of products S calculated by the above equation 5, based on the following equation 6, in order to eliminate deviation of the distribution of the probabilities of occurrence for each person. The value s obtained through normalization indicates the probability of the individual from which the walking vibration data targeted for determination was acquired being the respective individual corresponding to the individual specification data. [Equation 6] $s = S / \left(\sum {c}_{n}\right)$

For example, assume that the individual specification data is the data shown in FIGS. 9(a) to 9(d), and the binary data of the walking vibration data of the individual targeted for determination is the binary data shown in FIG. 11. In this case, the calculation results of the probability s by the individual determination unit 12 will be as follows. Accordingly, the individual determination unit 12 determines that the individual from which the walking vibration data targeted for determination was acquired is "person A". In this way, in the example embodiment, the individual is specified by contrasting the obtained binary data of walking vibration data with individual specification data, and verifying the similarity.
Person A: 0.786
Person B: 0.448
Person C: 0.000
Person D: 0.000

Also, the individual determination unit 12 may determine the individual from which the walking vibration data targeted for determination was acquired, by deriving the positions of the peaks in the binary data shown in FIG. 11, and contrasting the derived positions of the peaks with the table shown in FIG. 10.

### [Apparatus operations]

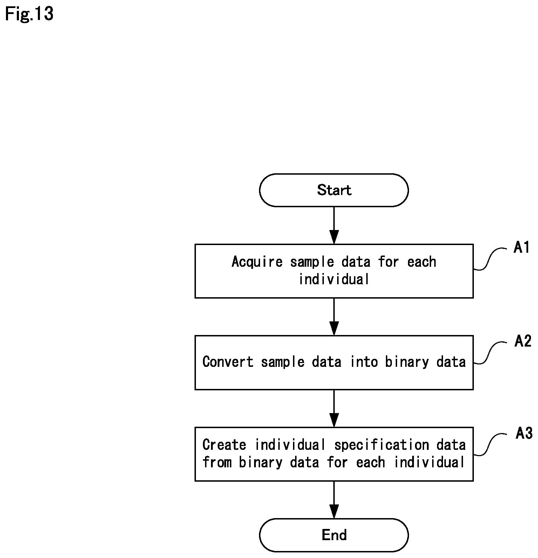

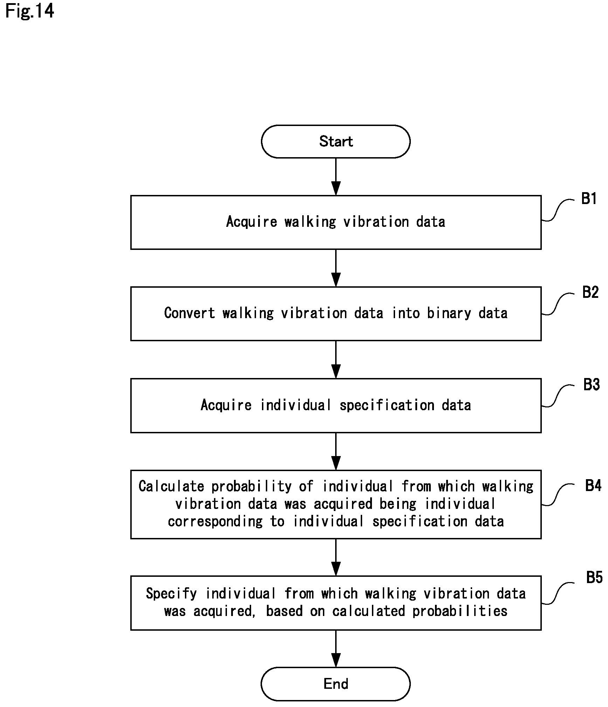

Next, the operations of the walking vibration analysis apparatus 10 according to the example embodiment will be described using FIGS. 13 and 14. In the following description, FIGS. 1 to 12 will be taken into consideration as appropriate. Also, in the example embodiment, the walking vibration analysis method is implemented by operating the walking vibration analysis apparatus 10. Therefore, description of the walking vibration analysis method according to the example embodiment will be replaced by the following description of the operations of the walking vibration analysis apparatus 10.

Initially, processing for creating individual specification data by the walking vibration analysis apparatus 10 will be described using FIG. 13. FIG. 13 is a flow diagram showing operations at the time of individual specification data creation processing by the walking vibration analysis apparatus according to the example embodiment.

As shown in FIG. 13, initially, the individual specification data creation unit 13 acquires, for each individual, a plurality of steps of walking vibration data of the individual as sample data (step A1). The sample data may be acquired from the vibration sensor 20, or may be input from another terminal device or the like.

Next, the individual specification data creation unit 13 converts, for each individual, the plurality of steps of sample data of the individual acquired in step A1 into binary data (step A2).

Next, the individual specification data creation unit 13 creates, for each individual, individual specification data from the binary data, and stores the created individual specification data in the data storage unit 14 (step A3).

Specifically, the individual specification data creation unit 13 composites, for each individual, the sample data using the abovementioned equation 3. The individual specification data creation unit 13 then creates high-level data from the sample data after composition, further using equation 4, and calculates the probability of occurrence of high-level portions. Thereafter, the individual specification data creation unit 13 stores, for each individual, the calculated probability of occurrence in the data storage unit 14 as the individual specification data of the individual.

Next, processing for specifying an individual by the walking vibration analysis apparatus 10 will be described using FIG. 14. FIG. 14 is a flow diagram showing operations at the time of individual specification processing by the walking vibration analysis apparatus according to the example embodiment.

As shown in FIG. 14, initially, the binarization processing unit 11 acquires sensor data output by the vibration sensor 20, as walking vibration data (step B1).

Next, the binarization processing unit 11 converts the walking vibration data acquired in step B1 into binary data (step B2). Also, the binarization processing unit 11 passes the binary data obtained through conversion to the individual determination unit 12.

Next, the individual determination unit 12, upon receiving the binary data, acquires the individual specification data of each individual created by the individual specification data creation unit 13 from the data storage unit 14 (step B3).

Next, the individual determination unit 12 contrasts, for each piece of individual specification data, high-level portions of the binary data acquired in step B2 with the probability of occurrence of high-level portions of the individual specification data acquired in step B3. The individual determination unit 12 then calculates the probability of the individual from which the walking vibration data was acquired being the individual corresponding to the individual specification data, based on the contrast results (step B4).

Thereafter, the individual determination unit 12 specifies the individual from which the walking vibration data acquired in step B1 was acquired, using the probabilities calculated in step B4 (step B5).

### [Effects of the Example Embodiment]

In this way, in the example embodiment, individual specification data is created, by superimposing a plurality of steps of walking vibration, and indexing the probability of a high-level portion occurring at respective timings. Because the probability of occurrence of high-level portions is a value unique to each individual, an individual can be specified by contrasting obtained binary data of walking vibration data with individual specification data. Thus, according to the example embodiment, an individual can be specified from walking vibration, even in the case where variation occurs in the walking vibration.

### [Program]

A program according to the example embodiment need only be a program that causes a computer to carry out steps A1 to A3 shown in FIG. 13 and steps B1 to B5 shown in FIG. 14. The walking vibration analysis apparatus 10 and the walking vibration analysis method according to the example embodiment can be realized, by this program being installing on a computer and executed. In this case, a processor of the computer performs processing while functioning as the binarization processing unit 11, the individual determination unit 12, and the individual specification data creation unit 13.

Also, the program according to the example embodiment may be executed by a computer system constructed from a plurality of computers. In this case, for example, each computer may respectively function as one of the binarization processing unit 11, the individual determination unit 12, and the individual specification data creation unit 13.

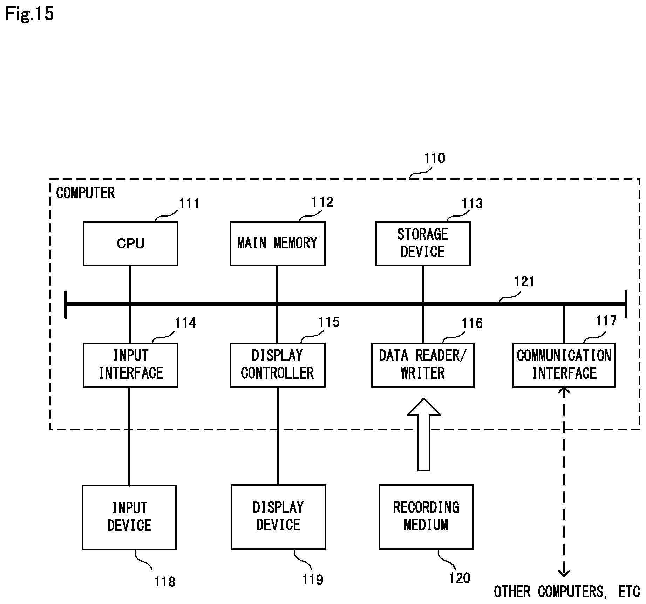

Here, a computer that realizes the walking vibration analysis apparatus 10 by executing a program according to the example embodiment will be described using FIG. 15. FIG. 15 is a block diagram showing an example of a computer that realizes the walking vibration analysis apparatus according to the example embodiment.

As shown in FIG. 15, a computer 110 includes a CPU (Central Processing Unit) 111, a main memory 112, a storage device 113, an input interface 114, a display controller 115, a data reader/writer 116, and a communication interface 117. These units are connected to each other in a manner that enables data communication, via a bus 121. Note that the computer 110 may include a GPU (Graphics Processing Unit) or an FPGA (Field-Programmable Gate Array), in addition to the CPU 111 or instead of the CPU 111.

The CPU 111 implements various computational operations, by extracting programs (code) according to the example embodiment that are stored in the storage device 113 to the main memory 112, and executing these programs in predetermined order. The main memory 112, typically, is a volatile storage device such as a DRAM (Dynamic Random Access Memory). Also, programs according to the example embodiment are provided in a state of being stored in a computer-readable recording medium 120. Note that programs according to the example embodiment may be distributed over the Internet connected via the communication interface 117.

Also, a semiconductor storage device such as a flash memory is given as a specific example of the storage device 113, other than a hard disk drive. The input interface 114 mediates data transmission between the CPU 111 and input devices 118 such as a keyboard and a mouse. The display controller 115 is connected to a display device 119 and controls display by the display device 119.

The data reader/writer 116 mediates data transmission between the CPU 111 and the recording medium 120, and executes readout of programs from the recording medium 120 and writing of processing results of the computer 110 to the recording medium 120. The communication interface 117 mediates data transmission between the CPU 111 and other computers.

Also, a general-purpose semiconductor storage device such as a CF (Compact Flash (registered trademark)) card or an SD (Secure Digital) card, a magnetic recording medium such as a flexible disk, and an optical recording medium such as a CD-ROM (Compact Disk Read Only Memory) are given as specific examples of the recording medium 120.

Note that the walking vibration analysis apparatus 10 according to the example embodiment is also realizable by using hardware corresponding to the respective units, rather than by a computer on which programs are installed. Furthermore, the walking vibration analysis apparatus 10 may be realized in part by programs, and the remaining portion may be realized by hardware.

### INDUSTRIAL APPLICABILITY

As described above, according to the example embodiments, an individual can be specified from walking vibration, even in the case where variation occurs in the walking vibration. The example embodiments is useful in systems that seek to specify individuals from walking vibration, such as systems that watch over elderly people and crime prevention systems, for example.

### LIST OF REFERENCE SIGNS

- 10: Walking vibration analysis apparatus
- 112: Binarization processing unit
- 12: Individual determination unit
- 13: Individual specification data creation unit
- 14: Data storage unit
- 20: Vibration sensor
- 21: Person
- 22: Building
- 110: Computer
- 111: CPU
- 112: Main memory
- 113: Storage device
- 114: Input interface
- 115: Display controller
- 116: Data reader/writer
- 117: Communication interface
- 118: Input device
- 119: Display device
- 120: Recording medium
- 121: Bus

## Claims

1. A walking vibration analysis apparatus (10) comprising:
a binarization processing unit (112) configured to acquire walking vibration data specifying walking vibration produced in association with walking, and convert the acquired walking vibration data into binary data representing a value of vibration over time from a start of walking with two values, being high level and low level;
an individual determination unit (12) configured to specify an individual from which the walking vibration data was acquired, by collating the binary data obtained through conversion with individual specification data created, for each individual in advance, by converting the walking vibration data of the individual into the binary data; and
an individual specification data creation unit (13) configured to create, for each of the individuals, the individual specification data of the individual,
wherein the individual specification data creation unit (13) is configured to acquire, for each of the individuals, a plurality of steps of walking vibration data of the individual (aₘₙ), wherein aₘₙ is the value of each piece of walking vibration data at any given point in time of an mth step, wherein m = 1 to M, and wherein 0 ≤ n ≤ N;
convert the acquired plurality of steps of walking vibration data into the binary data (bₘₙ), wherein m = 1 to M, and wherein 0 ≤ n ≤ N;
compose the converted plurality of steps of walking vibration data by calculating an average value (cₙ) for every piece of converted plurality of steps of walking vibration data whose n value is the same using the equation ${c}_{n} = \sum {b}_{mn} / M ,$ wherein 0 ≤ n ≤ N;
filter the composed converted plurality of steps of walking vibration data, using a threshold value; and
create the individual specification data of the individual using the filtered composed converted plurality of steps walking vibration data.

2. The walking vibration analysis apparatus (10) according to claim 1,
wherein the binarization processing unit (112) calculates a standard deviation of the acquired walking vibration data, and converts the acquired walking vibration data into the binary data, using the calculated standard deviation as the threshold value.

3. The walking vibration analysis apparatus (10) according to claim 1 or 2,
wherein the individual determination unit (12) contrasts, for each piece of the individual specification data, high-level portions of the binary data obtained through conversion with a probability of occurrence of high-level portions of the individual specification data, and calculates a probability of the individual from which the walking vibration data was acquired being the individual corresponding to the individual specification data.

4. A walking vibration analysis method comprising:
acquiring walking vibration data specifying walking vibration produced in association with walking, and converting the acquired walking vibration data into binary data representing a value of vibration over time from a start of walking with two values, being high level and low level;
specifying an individual from which the walking vibration data was acquired, by collating the binary data obtained through conversion with individual specification data created, for each individual in advance, by converting the walking vibration data of the individual into the binary data; and
creating, for each of the individuals, the individual specification data of the individual, wherein, in the creating, for each of the individuals, a plurality of steps of walking vibration data of the individual (aₘₙ) are acquired, wherein aₘₙ is the value of each piece of walking vibration data at any given point in time of an mth step, wherein m = 1 to M, and wherein 0 ≤ n ≤ N;
the acquired plurality of steps of walking vibration data are converted into the binary data (bₘₙ), wherein m = 1 to M, and wherein 0 ≤ n ≤ N;
the converted plurality of steps of walking vibration data are composed by calculating an average value (cₙ) for every piece of converted plurality of steps of walking vibration data whose n value is the same using the equation ${c}_{n} = \sum {b}_{mn} / M ,$ wherein 0 ≤ n ≤ N;
the composed converted plurality of steps of walking vibration data are filtered using a threshold value; and
the individual specification data of the individual is created using the filtered composed converted plurality of steps walking vibration data.

5. The walking vibration analysis method according to claim 4,
wherein, in the acquiring, a standard deviation of the acquired walking vibration data is calculated, and the acquired walking vibration data is converted into the binary data, using the calculated standard deviation as the threshold value.

6. The walking vibration analysis method according to claim 4 or 5,
wherein, in the specifying, for each piece of the individual specification data, high-level portions of the binary data obtained through conversion are contrasted with a probability of occurrence of high-level portions of the individual specification data, and a probability of the individual from which the walking vibration data was acquired being the individual corresponding to the individual specification data is calculated.

7. A program including instructions that cause the computer to execute the method of any of claims 4 to 6.

## Patentansprüche

1. Vorrichtung zur Analyse von Laufschwingungen (10), die aufweist:
eine Digitalisierungsverarbeitungseinheit (112), die konfiguriert ist, um Laufschwingungsdaten zu erlangen, welche die in Verbindung mit dem Laufen erzeugte Laufschwingung spezifizieren, und um die erlangten Laufschwingungsdaten in digitale Daten umzuwandeln, die einen Wert der Schwingung über die Zeit ab einem Start des Laufens mit zwei Werten darstellen, welche der hohe Pegel und der niedrige Pegel sind;
eine Personenbestimmungseinheit (12), die konfiguriert ist, um eine Person zu spezifizieren, von welcher die Laufschwingungsdaten erlangt wurden, indem die durch Umwandlung gewonnenen digitalen Daten mit persönlichen Spezifikationsdaten verglichen werden, die für jede Person im Voraus erzeugt werden, indem die Laufschwingungsdaten für die Person in die digitalen Daten umgewandelt werden; und
eine Personenspezifikationsdaten-Erzeugungseinheit (13), die konfiguriert ist, um für jede der Personen die Personenspezifikationsdaten der Person zu erzeugen,
wobei die Personenspezifikationsdaten-Erzeugungseinheit (13) konfiguriert ist, um für jede der Personen mehrere Schritte von Laufschwingungsdaten der Person (aₘₙ) zu erlangen, wobei aₘₙ der Wert jedes Laufschwingungsdatenelements zu einem beliebigen Zeitpunkt eines m-ten Schritts ist, wobei m = 1 bis M und wobei 0 ≤ n ≤ N;
um die erlangten mehreren Schritte von Laufschwingungsdaten in die digitalen Daten (bₘₙ) umzuwandeln, wobei m = 1 bis M und wobei 0 ≤ n ≤ N;
um die umgewandelten mehreren Schritte von Laufschwingungsdaten zusammenzusetzen, indem ein Durchschnittswert (cₙ) für jedes einzelne Element der umgewandelten mehreren Schritte von Laufschwingungsdaten, dessen n-Wert gleich ist, unter Verwendung der folgenden Gleichung berechnet wird ${c}_{n} = \sum {b}_{mn} / M , wobei 0 \leq n \leq N ;$
um die zusammengesetzten umgewandelten mehreren Schritte von Laufschwingungsdaten unter Verwendung eines Schwellwerts zu filtern; und
die Personenspezifikationsdaten der Person unter Verwendung der gefilterten zusammengesetzten umgewandelten mehreren Schritte von Laufschwingungsdaten zu erzeugen.

2. Vorrichtung zur Analyse von Laufschwingungen (10) nach Anspruch 1,
wobei die Digitalisierungsverarbeitungseinheit (112) eine Standardabweichung der erlangten Laufschwingungsdaten berechnet und die erlangten Laufschwingungsdaten unter Verwendung der berechneten Standardabweichung als der Schwellwert in die digitalen Daten umwandelt.

3. Vorrichtung zur Analyse von Laufschwingungen (10) nach Anspruch 1 oder 2,
wobei die Personenbestimmungseinheit (12) für jedes der Personenspezifikationsdatenelemente Abschnitte der durch Umwandlung gewonnenen digitalen Daten mit hohem Pegel einer Auftrittswahrscheinlichkeit für Abschnitte mit hohem Pegel der Personenspezifikationsdaten gegenüberstellt und eine Wahrscheinlichkeit der Person, von der die Laufschwingungsdaten erlangt wurden, welche die Person ist, die den Personenspezifikationsdaten entspricht, berechnet.

4. Verfahren zur Laufschwingungsanalyse, das aufweist:
Erlangen von Laufschwingungsdaten, welche die in Verbindung mit dem Laufen erzeugte Laufschwingung spezifizieren, und Umwandeln der erlangten Laufschwingungsdaten in digitale Daten, die einen Wert der Schwingung über die Zeit ab einem Start des Laufens mit zwei Werten darstellen, welche der hohe Pegel und der niedrige Pegel sind;
Spezifizieren einer Person, von welcher die Laufschwingungsdaten erlangt wurden, indem die durch Umwandlung gewonnenen digitalen Daten mit persönlichen Spezifikationsdaten verglichen werden, die für jede Person im Voraus erzeugt werden, indem die Laufschwingungsdaten für die Person in die digitalen Daten umgewandelt werden; und
für jede der Personen Erzeugen der Personenspezifikationsdaten der Person,
wobei bei der Erzeugung für jede der Personen mehrere Schritte von Laufschwingungsdaten der Person (aₘₙ) erlangt werden, wobei aₘₙ der Wert jedes Laufschwingungsdatenelements zu einem beliebigen Zeitpunkt eines m-ten Schritts ist, wobei m = 1 bis M und wobei 0 ≤ n ≤ N;
die erlangten mehreren Schritte von Laufschwingungsdaten in die digitalen Daten (bₘₙ) umgewandelt werden, wobei m = 1 bis M und wobei 0 ≤ n ≤ N;
die umgewandelten mehreren Schritte von Laufschwingungsdaten zusammengesetzt werden, indem ein Durchschnittswert (cₙ) für jedes einzelne Element der umgewandelten mehreren Schritte von Laufschwingungsdaten, dessen n-Wert gleich ist, unter Verwendung der folgenden Gleichung berechnet wird ${c}_{n} = \sum {b}_{mn} / M , wobei 0 \leq n \leq N ;$
die zusammengesetzten umgewandelten mehreren Schritte von Laufschwingungsdaten unter Verwendung eines Schwellwerts gefiltert werden; und
die Personenspezifikationsdaten der Person unter Verwendung der gefilterten zusammengesetzten umgewandelten mehreren Schritte von Laufschwingungsdaten erzeugt werden.

5. Verfahren zur Laufschwingungsanalyse nach Anspruch 4,
wobei bei der Erlangung eine Standardabweichung der erlangten Laufschwingungsdaten berechnet wird und die erlangten Laufschwingungsdaten unter Verwendung der berechneten Standardabweichung als der Schwellwert in die digitalen Daten umwandelt werden.

6. Verfahren zur Laufschwingungsanalyse nach Anspruch 4 oder 5,
wobei bei der Spezifizierung für jedes der Personenspezifikationsdatenelemente Abschnitte der durch Umwandlung gewonnenen digitalen Daten mit hohem Pegel einer Auftrittswahrscheinlichkeit für Abschnitte mit hohem Pegel der Personenspezifikationsdaten gegenübergestellt werden und eine Wahrscheinlichkeit der Person, von der die Laufschwingungsdaten erlangt wurden, welche die Person ist, die den Personenspezifikationsdaten entspricht, berechnet wird.

7. Programm, das Anweisungen aufweist, die bewirken, dass der Computer das Verfahren nach einem der Ansprüche 4 bis 6 ausführt.

## Revendications

1. Appareil d'analyse de vibrations de marche (10) comprenant :
une unité de traitement de binarisation (112) configurée pour acquérir des données de vibrations de marche spécifiant des vibrations de marche produites en association avec la marche, et convertir les données de vibrations de marche acquises en données binaires représentant une valeur de vibration au fil du temps à partir d'un début de marche avec deux valeurs, à savoir un niveau haut et un niveau bas ;
une unité de détermination d'individu (12) configurée pour spécifier un individu duquel les données de vibrations de marche ont été acquises, en collationnant les données binaires obtenues par conversion avec des données de spécification d'individu créées, pour chaque individu à l'avance, en convertissant les données de vibrations de marche de l'individu en lesdites données binaires ; et
une unité de création de données de spécification d'individu (13) configurée pour créer, pour chacun des individus, les données de spécification d'individu de l'individu,
dans lequel l'unité de création de données de spécification d'individu (13) est configurée pour acquérir, pour chacun des individus, une pluralité de phases de données de vibrations de marche de l'individu (aₘₙ), où aₘₙ est la valeur de chaque élément de données de vibrations de marche à un instant donné quelconque d'une m-ième phase, où m = 1 à M, et où 0 ≤ n ≤ N ;
convertir la pluralité acquise de phases de données de vibrations de marche en lesdites données binaires (bₘₙ), où m = 1 à M, et où 0 ≤ n ≤ N ;
composer la pluralité convertie de phases de données de vibrations de marche en calculant une valeur moyenne (cₙ) pour chaque élément de la pluralité convertie de phases de données de vibrations de marche dont la valeur n est la même en utilisant l'équation ${c}_{n} = {Σb}_{mn} / M , où 0 \leq n \leq N ;$
filtrer la pluralité convertie et composée de phases de données de vibrations de marche, en utilisant une valeur de seuil ; et
créer les données de spécification d'individu de l'individu en utilisant la pluralité convertie, composée et filtrée de phases de données de vibrations de marche.

2. Appareil d'analyse de vibrations de marche (10) selon la revendication 1,
dans lequel l'unité de traitement de binarisation (112) calcule un écart type des données de vibrations de marche acquises, et convertit les données de vibrations de marche acquises en lesdites données binaires, en utilisant l'écart type calculé comme valeur de seuil.

3. Appareil d'analyse de vibrations de marche (10) selon la revendication 1 ou 2,
dans lequel l'unité de détermination d'individu (12) compare, pour chaque élément des données de spécification d'individu, des portions de niveau haut des données binaires obtenues par conversion à une probabilité d'occurrence de portions de niveau haut des données de spécification d'individu, et calcule une probabilité que l'individu duquel les données de vibrations de marche ont été acquises soit l'individu correspondant aux données de spécification d'individu.

4. Procédé d'analyse de vibrations de marche comprenant :
l'acquisition de données de vibrations de marche spécifiant des vibrations de marche produites en association avec la marche, et la conversion des données de vibrations de marche acquises en données binaires représentant une valeur de vibration au fil du temps à partir d'un début de marche avec deux valeurs, à savoir un niveau haut et un niveau bas ;
la spécification d'un individu duquel les données de vibrations de marche ont été acquises, en collationnant les données binaires obtenues par conversion avec des données de spécification d'individu créées, pour chaque individu à l'avance, en convertissant les données de vibrations de marche de l'individu en lesdites données binaires ; et
la création, pour chacun des individus, des données de spécification d'individu de l'individu,
dans lequel, dans la création, pour chacun des individus, une pluralité de phases de données de vibrations de marche de l'individu (aₘₙ) est acquise, où aₘₙ est la valeur de chaque élément de données de vibrations de marche à un instant donné quelconque d'une m-ième phase, où m = 1 à M, et où 0 ≤ n ≤ N ;
la pluralité acquise de phases de données de vibrations de marche est convertie en lesdites données binaires (bₘₙ), où m = 1 à M, et où 0 ≤ n ≤ N ;
la pluralité convertie de phases de données de vibrations de marche est composée en calculant une valeur moyenne (cₙ) pour chaque élément de la pluralité convertie de phases de données de vibrations de marche dont la valeur n est la même en utilisant l'équation ${c}_{n} = {Σb}_{mn} / M , où 0 \leq n \leq N ;$
la pluralité convertie et composée de phases de données de vibrations de marche est filtrée en utilisant une valeur de seuil ; et
les données de spécification d'individu de l'individu sont créées en utilisant la pluralité convertie, composée et filtrée de phases de données de vibrations de marche.

5. Procédé d'analyse de vibrations de marche selon la revendication 4,
dans lequel, dans l'acquisition, un écart type des données de vibrations de marche acquises est calculé, et les données de vibrations de marche acquises sont converties en lesdites données binaires, en utilisant l'écart type calculé comme valeur de seuil.

6. Procédé d'analyse de vibrations de marche selon la revendication 4 ou 5,
dans lequel, dans la spécification, pour chaque élément des données de spécification d'individu, des portions de niveau haut des données binaires obtenues par conversion sont comparées à une probabilité d'occurrence de portions de niveau haut des données de spécification d'individu, et une probabilité que l'individu duquel les données de vibrations de marche ont été acquises soit l'individu correspondant aux données de spécification d'individu est calculée.

7. Programme comprenant des instructions qui amènent l'ordinateur à exécuter le procédé selon l'une quelconque des revendications 4 à 6.
